# EUROPEAN PATENT APPLICATION

(11) **EP 4 233 814 A2**
(43) Date of publication of application: **30.08.2023**
(21) Application number: 23175811.1
(22) Date of filing: 12.06.2018
(51) Int. Cl.: A61F 13/02

(54) **FABRIC WOUND FILLER**

(30) Priority: 12.06.2017 US 201762518283 P
(62) Divisional of application: 18735162.2
(71) Applicant: 3M Innovative Properties Company, Saint Paul, MN 55133-3427 (US)
(72) Inventor: ROBINSON, Timothy, Mark, Shillingstone, DT11 0TG (GB); LOCKE, Christopher Brian, Bournemouth, BH9 3SD (GB); ALLEN, Diwi L., San Antonio, TX, 78255 (US); LONG, Justin Alexander, Bournemouth (GB); COURAGE, James, San Antonio, TX (US)
(74) Representative: Simmons & Simmons

(57) **Abstract**

A method may include forming a mesh comprising a plurality of strands meshed together. Each of the plurality of strands may include a polymeric film. The method may include perforating the mesh to form a perforated mesh having one or more perforations. The method may further include texturing the perforated mesh to form at textured and perforated mesh having at least one of a dimple or a channel. A system may include a cover configured to form a seal over the tissue site. The system may also include a reduced-pressure source configured to provide reduced pressure through the cover at the tissue site. The system may further include a tissue interface configured to be disposed underneath the cover at the tissue site. The tissue interface may include a textured mat having a plurality of strands of polymeric film matted together and at least one of a dimple or a channel.

## Description

### RELATED APPLICATION

This application claims the benefit, under 35 U.S.C. § 119(e), of the filing of U.S. Provisional Patent Application serial number 62/518,283, entitled "FABRIC WOUND FILLER," filed June 12, 2017, which is incorporated herein by reference for all purposes.

### TECHNICAL FIELD

The invention set forth in the appended claims relates generally to tissue treatment systems and more particularly, but without limitation, to making and using a manifolding wound filler for use in conjunction with reduced pressure wound therapy.

### BACKGROUND

Clinical studies and practice have shown that reducing pressure in proximity to a tissue site may augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "reduced-pressure therapy," but is also known by other names, including "negative-pressure wound therapy," "negative-pressure therapy," "vacuum therapy," "vacuum-assisted closure," and "topical negative-pressure," for example. Reduced-pressure therapy may provide a number of benefits, including migration of epithelial and subcutaneous tissues, improved blood flow, and micro-deformation of tissue at a wound site. Together, these benefits may increase development of granulation tissue and reduce healing times.

Tissue treatment systems may use a tissue interface (such as a wound manifold or a wound filler) that when installed at a tissue site (such as on a wound or in a wound at a tissue site) facilitates various degrees of tissue ingrowth that may cause pain or discomfort to a patient and in some cases leave remnants of the tissue interface in the wound when the tissue interface is removed from the tissue site. Accordingly, improvements to the tissue interface are desirable.

While the clinical benefits of reduced-pressure therapy and tissue interfaces are widely known, improvements to therapy systems, components, and processes may benefit healthcare providers and patients.

### BRIEF SUMMARY

New and useful systems, apparatuses, and methods for manufacturing and implementing tissue interfaces with embossed meshed films and embossed manifolding wound fillers are set forth in the appended claims. Illustrative embodiments are also provided to enable a person skilled in the art to make and use the claimed subject matter.

In a first embodiment, a method of manufacturing a tissue interface for a reduced-pressure tissue treatment system is provided. The method may include forming a spacer fabric comprising a plurality of fibers. Each of the plurality of fibers includes a polymeric material. The method may also include fusing the plurality of fibers of the spacer fabric together. The method may further include applying a hydrophilic material to the plurality of fibers.

In a second embodiment, a method of manufacturing a tissue interface for a reduced pressure tissue treatment system is provided. The method may include forming a mesh comprising a plurality of strands meshed together. Each of the plurality of strands may include a polymeric film. The method may also include perforating the mesh to form a perforated mesh having one or more perforations. The method may further include texturing the perforated mesh to form at textured and perforated mesh having at least one of a dimple or a channel.

In a third embodiment, a system to provide reduced pressure at a tissue site. The system may include a cover configured to form a seal over the tissue site. The system may also include a reduced-pressure source configured to provide reduced pressure through the cover at the tissue site. The system may further include a tissue interface configured to be disposed underneath the cover at the tissue site. The tissue interface may include a textured mat having a plurality of strands of polymeric film matted together and at least one of a dimple or a channel.

In a fourth embodiment, a tissue interface configured to be disposed at a tissue site is provided. The tissue interface may include a textured and perforated mat. The textured and perforated mat may include a plurality of matted strands of polymeric film. At least one strand of the plurality of strands may include a polymeric film that includes a material that is different from a material of a polymeric film of at least one other strand of the plurality of strands. The textured and perforated mat may also include at least one of a dimple or a channel formed by texturing a surface of the mat to form a textured mat. The textured and perforated mat may further include one or more perforations formed by perforating the textured mat to form the textured and perforated mat. A shape of the one or more perforations may include at least one of a circle, a polygon, or an oval. A diameter of the one or more perforations may be between approximately 0.5 mm and approximately 1 mm. The tissue interface may be configured to be disposed underneath a cover at the tissue site. The tissue interface may also be configured to receive reduced pressure from a reduced pressure source.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a simplified functional block diagram of an example embodiment of a therapy system for providing reduced-pressure therapy comprising a tissue interface including an embossed mesh film or an embossed manifolding wound filler;
Figure 2 is a top view illustrating a tissue interface of the therapy system of Figure 1;
Figure 3 is a top view illustrating a tissue interface of the therapy system of Figure 1;
Figure 4 is a flow chart of a method for manufacturing a fabric of a tissue interface for the therapy system of Figure 1;
Figure 5 is a perspective schematic view illustrating a first embodiment of a tissue interface comprising a plurality of fibers for the therapy system of Figure 1;
Figure 6A is a perspective view of a first embodiment of at least some of the plurality of fibers of the tissue interface of Figure 5 contacting each other;
Figure 6B is a perspective view of a second embodiment of at least some of the plurality of fibers of the tissue interface of Figure 5 coupled to each other;
Figure 7 is a cross-sectional view illustrating details that may be associated with some example embodiments of the plurality of fibers of Figures 5, 6A, and 6B;
Figure 8 is a perspective schematic view illustrating a second embodiment of a tissue interface for the therapy system of Figure 1; and
Figure 9 is a flow chart of a second method of manufacturing a tissue interface for the therapy system of Figure 1.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

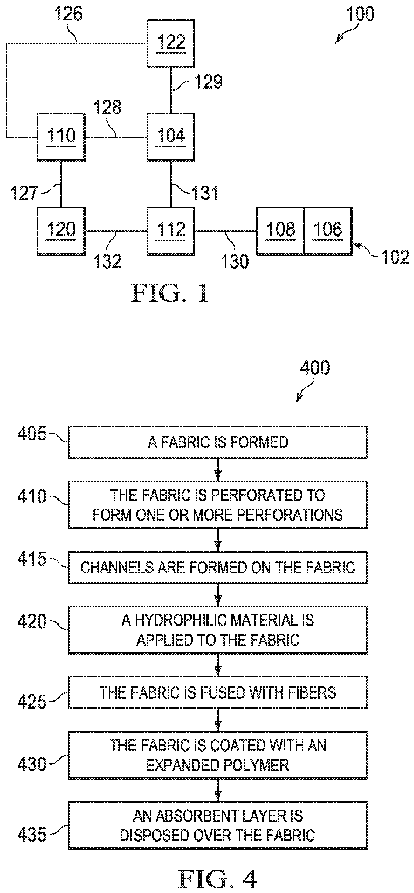

Figure 1 is a simplified functional block diagram of an example embodiment of a therapy system 100 for providing reduced-pressure therapy comprising a tissue interface including an embossed mesh film or an embossed, manifolding wound filler.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, reduced pressure may be applied to a tissue site to grow additional tissue that may be harvested and transplanted.

The therapy system 100 may include reduced-pressure supply, and may include or be configured to be coupled to a distribution component or tubing, such as a dressing. In general, a distribution component may refer to any complementary or ancillary component configured to be fluidly coupled to a reduced-pressure supply in a fluid path between a reduced-pressure supply and a tissue site. A distribution component is preferably detachable, and may be disposable, reusable, or recyclable. For example, a dressing 102 may be fluidly coupled to a reduced-pressure source 104, as illustrated in Figure 1. A dressing may include a cover, a tissue interface, or both in some embodiments. The dressing 102, for example, may include a cover 106 and a tissue interface 108. A regulator or a controller, such as a controller 110, may also be coupled to the reduced-pressure source 104.

In some embodiments, a dressing interface may facilitate coupling the reduced-pressure source 104 to the dressing 102. For example, such a dressing interface may be a T.R.A.C.^{®} Pad or Sensa T.R.A.C.^{®} Pad available from KCI of San Antonio, Texas. The therapy system 100 may optionally include a fluid container, such as a container 112, coupled to the dressing 102 and to the reduced-pressure source 104.

Additionally, the therapy system 100 may include sensors to measure operating parameters and provide feedback signals to the controller 110 indicative of the operating parameters. As illustrated in Figure 1, for example, the therapy system 100 may include at least one of a pressure sensor 120 or an electric sensor 122 coupled to the controller 110 via electric conductors 126 and 127, respectively. The pressure sensor 120 may also be coupled or configured to be fluidly coupled via a distribution component such as, for example, fluid conduits 131 and 132 and to the reduced-pressure source 104. For example, as shown in Figure 1, the electric conductor 126 provides electric communication between the electric sensor 122 and the controller 110. The electric conductor 127 provides electric communication between the pressure sensor 120 and the controller 110. The electric conductor 128 provides electric communication between the controller 110 and the reduced-pressure source 104. The electric conductor 129 provides electric communication between the electric sensor 122 and the reduced-pressure source 104.

Components may be fluidly coupled to each other to provide a path for transferring fluids (such as at least one of liquid or gas) between the components. Components may be fluidly coupled through a fluid conductor, such as a tube. For example fluid conductor 130 provides fluid communication between the dressing 102 and the container 112; fluid conductor 131 provides fluid communication between the reduced-pressure source 104 and the container 112; and fluid conductor 132 provides fluid communication between the pressure sensor 120 and the container 112. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors 124 may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the reduced-pressure source 104 may be directly coupled to the controller 110, and may be indirectly coupled to the dressing 102 through the container 112.

The fluid mechanics of using a reduced-pressure source to reduce pressure in another component or location, such as within a sealed therapeutic environment, can be mathematically complex. However, the basic principles of fluid mechanics applicable to reduced-pressure therapy are generally well-known to those skilled in the art, and the process of reducing pressure may be described illustratively herein as "delivering," "distributing," or "generating" reduced pressure, for example.

In general, exudates and other fluids flow toward lower pressure along a fluid path. Thus, the term "downstream" typically implies something in a fluid path relatively closer to a source of reduced pressure or further away from a source of positive pressure. Conversely, the term "upstream" implies something relatively further away from a source of reduced pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a reduced-pressure source) and this descriptive convention should not be construed as a limiting convention.

"Reduced pressure" or "negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in reduced pressure typically refer to a decrease in absolute pressure, while decreases in reduced pressure typically refer to an increase in absolute pressure. While the amount and nature of reduced pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A reduced-pressure supply, such as the reduced-pressure source 104, may be a reservoir of air at a reduced pressure, or may be a manual or electrically-powered device that can reduce the pressure in a sealed volume, such as a vacuum pump, a suction pump, a wall suction port available at many healthcare facilities, or a micro-pump, for example. A reduced-pressure supply may be housed within or used in conjunction with other components, such as sensors, processing units, alarm indicators, memory, databases, software, display devices, or user interfaces that further facilitate therapy. For example, in some embodiments, the reduced-pressure source 104 may be combined with the controller 110 and other components into a therapy unit. A reduced-pressure supply may also have one or more supply ports configured to facilitate coupling and de-coupling the reduced-pressure supply to one or more distribution components.

Sensors, such as the pressure sensor 120 or the electric sensor 122, are generally known in the art as any apparatus operable to detect or measure a physical phenomenon or property, and generally provide a signal indicative of the phenomenon or property that is detected or measured. For example, the pressure sensor 120 and the electric sensor 122 may be configured to measure one or more operating parameters of the therapy system 100. In some embodiments, the pressure sensor 120 may be a transducer configured to measure pressure in a pneumatic pathway and convert the measurement to a signal indicative of the pressure measured. In some embodiments, for example, the pressure sensor 120 may be a piezoresistive strain gauge. The electric sensor 122 may optionally measure operating parameters of the reduced-pressure source 104, such as the voltage or current, in some embodiments. Preferably, the signals from the pressure sensor 120 and the electric sensor 122 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 may be representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with reduced-pressure therapy.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a reduced pressure at a tissue site for a given reduced-pressure source. The cover 106 may have a high moisture-vapor transmission rate (MVTR) in some applications. For example, the MVTR may be at least 300 g/m^2 per twenty-four hours in some embodiments. In some example embodiments, the cover 106 may be a polymer drape, such as a polyurethane film, that is permeable to water vapor but impermeable to liquid. Such drapes typically have a thickness in the range of 25-50 µm (microns). For permeable materials, the permeability generally should be low enough that a desired reduced pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The tissue interface 108 may be configured to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive reduced pressure from a source and distribute reduced pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In operation, referring to at least Figure 1, the tissue interface 108 may be placed within, over, on, or otherwise proximate to a tissue site. The cover 106 may be placed over the tissue interface 108 and sealed to an attachment surface that may be near, around, or adjacent tothe tissue site. For example, the cover 106 may be sealed to undamaged epidermis peripheral to a tissue site. Thus, the dressing 102 may provide a sealed therapeutic environment proximate to a tissue site, substantially isolated from the external environment, and the reduced-pressure source 104 may reduce the pressure in the sealed therapeutic environment. Reduced pressure applied across the tissue site through the tissue interface 108 in the sealed therapeutic environment may induce macrostrain and microstrain in the tissue site, as well as remove exudates and other fluids from the tissue site, which can be collected in container 112.

The tissue interface 108 may comprise one or more fabrics that have a variety of shapes including, for example, fabrics having a sheet-like shape or thicker fabrics having more of a three-dimensional shape. Sheet-like shapes may include shapes where a portion of the shape is thin in comparison to its length or its width or breadth. Sheets may include meshes or mats. A mesh may include a woven, knit, or knotted material of open texture with evenly spaced holes. A mesh may also include a web-like pattern or construction. A mesh may further include an interlaced structure. Mats may include a tangled and sometimes thick mass of material that in at least some cases is pressed. Mats may also include a densely felted material.

Three-dimensional fabrics have a variety of geometric shapes such as circular shapes or rectangular shapes, or a variety of irregular shapes. The fabrics may possess various characteristics such as, for example, non-shedding fabrics, elastic fabrics, hydrophobic fabrics, and hydrophilic fabrics. An example embodiment of a three-dimensional fabric may include 3MESH^{™} available from MULLER^{™}. The fabrics may serve a variety of functions such as, for example, a manifold fabric as described above, a space fabric, a spacer fabric, a filler, or a combination of several functions. In some embodiments, three-dimensional fabrics may have a thickness between about 1.0 mm (millimeters) and about 50 mm. The tissue interface 108 may comprise one or more layers of the same or different fabrics that may be stacked together, laminated to each other, welded together, or sealed together by a variety of different adhesives.

Three-dimensional fabrics may include spacer fabrics. Spacer fabrics may include a knitted fabric consisting of two separate knitted substrates that are joined together or kept apart by a spacer yarn. Spacer fabrics may also include a first hydrophilic layer, a second hydroscopic layer, and a spacer layer having monofilaments or multifilaments. An example embodiment of a spacer fabric may include HEATHCOAT^{™}, and XD SPACER^{™} available from BALTEX^{™}.

As described above, the fabrics may include an elastic material that may be elastic in compression or expansion. For example, a fabric may be compressed or stretched in different directions and return to its initial shape and size when compression or stretching ceases. In some embodiments, the fabrics may be expanded to a fixed shape forming a three-dimensional structure such as a scaffold. The fabric may serve as a scaffold to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials. In various embodiments, the fabrics may be constructed from bioresorbable materials. When the fabrics are constructed from bioresorbable materials, if a portion of a fabric is left within the tissue site after removal of the tissue interface 108, the portion of the fabric may not cause infection or irritation at the tissue site. Suitable bioresorbable materials may include, without limitation, a polymeric blend of polylactic acid (PLA) and polyglycolic acid (PGA). A polymeric blend may also include without limitation at least one of polycarbonates, polyfumarates, or capralactones.

In at least some embodiments, the fabrics may be a treated fabric. For example, the fabrics may be treated or coated with a hydrophilic material or an elastic film. A treatment may include coating or impregnating a fabric with a hydrophilic material or adhering a hydrophilic material to the fabric. In some embodiments, the hydrophilic material or elastic film may be applied to the fabric in a specific pattern so that some portions of fabric are treated while other portions of the fabric are not treated. An elastic film may include a thermoplastic elastomer (TPE), a thermoplastic vulcanizates (TPV), thermoplastic polyurethane (TPU), or a polyether block amide (PEBAX). The fabric may be treated with a hydrophilic material or an elastic film to provide at least one of fluid transport, wicking, or absorbent properties. For example, a fabric of the tissue interface 108 treated with a hydrophilic material or an elastic film may allow the tissue interface 108 to wick fluid away from a tissue site, while continuing to distribute reduced pressure to the tissue site.

In some embodiments, the fabric may have a textured surface. For example, the fabric may be textured or treated to form a textured surface having an uneven, coarse, jagged, or other profile that may be capable of inducing microstrains and/or stresses at a tissue site when reduced pressure is applied to the fabric. The textured surface of the fabric may promote granulation at a tissue site when pressure within the sealed therapeutic environment is reduced or modulated causing some motion of the textured surface of the fabric adjacent the tissue site.

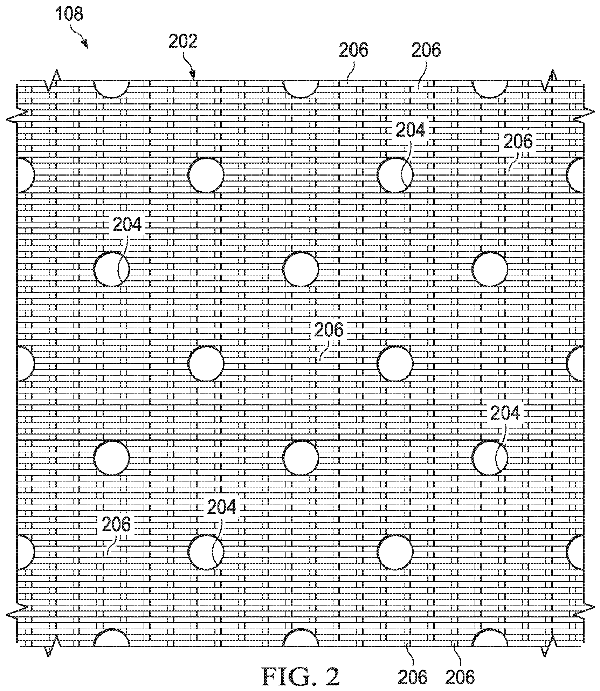

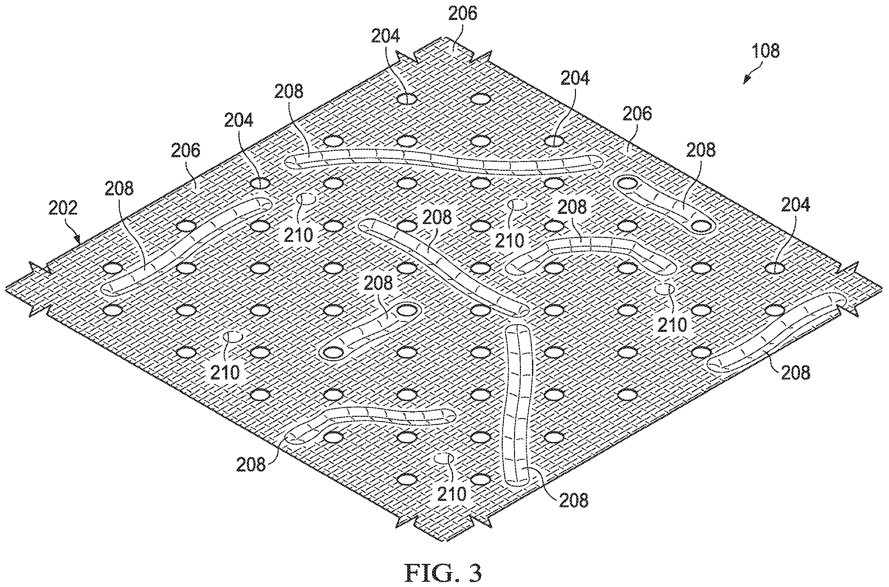

As indicated above, the fabrics may comprise a framework or structure in the form of a mesh or a mat that comprises a plurality of strands or fibers woven or knitted together to form the fabric. Referring more specifically to Figures 2 and 3, the tissue interface 108 comprises a fabric 202 formed by a plurality of strands or fibers 206 that are woven together in a perpendicular pattern to form the fabric 202. The fibers 206 may be coupled together using, for example, a bond, such as a weld or a glue. In some embodiments, the fibers 206 may be fused together, using, for example, a chemical fusing or heat fusing. Each of the fibers 206 may have a cross-sectional shape including a circle, a polygon, an oval, or other geometric shapes. In some embodiments, the fibers 206 may have a rectangular cross-section that is relatively flat to form, for example, a mat. One of ordinary skill in the art would be able to identify the various types of cross-sectional shapes that fibers needed to form the type of fabric that may be desired for a tissue interface 108 necessary for treating a tissue site.

The fibers 206 may be formed by extracting them from one or more yarns or one or more polymeric materials. For example, the fibers 206 may be formed of various different types of yarns or various types of polymeric materials. Polymeric materials may include at least one of a non-foam material or a non-woven material. One of ordinary skill in the art would be able to identify the type of yarn and polymeric materials that would be best suited for the fabric 202. Working examples of polymeric films include DELNET^{®} Apertured Films and STRATEX^{®} Engineered Composites from DelStar Technologies, Inc. of Middletown, Delaware. In some embodiments, a polymeric film may include a thermoplastic elastomer (TPE), a thermoplastic vulcanizates (TPV), a thermoplastic polyurethane (TPU), or a polyether block amide (PEBAX).

In various example embodiments, the fabric 202 may be perforated or textured to enhance would therapy. The fabric 202 may include indentations or hollows in the surface of the fabric 202. The fabric 202 may be perforated to include one or more fenestrations or perforations 204 extending through any of the embodiments of the fabric 202 described above. The perforations 204 may communicate fluid through the fabric 202 to enhance wound therapy including negative-pressure wound therapy. The fabric 202 may also include a surface having one or more indentations or hollows on the surface that do not extend through the fabric 202. Such indentations or hollows may have a variety of shapes such as, for example, one or more channels 208 or dimples 210 as shown more specifically in Figure 3.

In some embodiments, the fibers 206 forming the fabric 202 of the tissue interface 108 may be loosely woven to leave voids or gaps between the individual fibers 206. The gaps between the fibers 206 may be formed uniformly or randomly through the fabric 202 to facilitate fluid communication through the tissue interface in a fashion similar to the perforations 204 when, for example, reduced-pressure is applied to the tissue interface 108. In some embodiments, the gaps may be formed so that they are more concentrated in one region of the tissue interface 108 to enhance the application of reduced-pressure, while being less concentrated in other regions of the tissue interface 108. In some embodiments of the fabric 202, the gaps may be formed through the fabric 202 in combination with one or more of the other features described herein. For example, the gaps may be formed in the fabric 202 between, or in conjunction with, the dimples 210 and/or the channels 208.

In any embodiments of the tissue interface 108, the fabric 202 may be perforated so that one or more fenestrations or perforations 204 extend through the fabric 202. The perforations 204 may communicate fluid to the fabric 202 to enhance wound therapy including negative pressure wound therapy. The perforations 204 may be formed by punching holes through the fabric 202, perforating the fabric 202, cutting holes in the fabric 202, or any number of different methods. The perforations 204 may also be formed by vacuum forming methods. The perforations 204 may be formed into one or more shapes including, for example, an elliptical shape including a circle, a polygon, an oval, or a simple slit through the material. Perforations 204 having an elliptical shape may have diameters between about 300 µm and about 1000 µm. The perforations 204 may be stretched in different directions to change the shape of the perforations 204. Stretching the fabric 202 in one or more directions to form different shapes and sizes facilitates fluid communication through the fabric 202. The perforations 204 may be positioned randomly throughout the fabric 202. In at least some embodiments, the perforations 204 may be positioned so that one or more perforations 204 are more concentrated in one region of the fabric 202 while other perforations 204 are less concentrated in other regions of the fabric 202. In some embodiments of the tissue interface 108, the fabric 202 may include perforations 204 that are formed through the fabric 202 in combination with one or more of the other features described herein. For example, the perforations 204 may be through-holes extending through the fabric 202 from the base of channels discussed herein rather than or in addition to a surface of the fabric 202.

Any embodiments of the tissue interface 108 described herein, the fabric 202 may additionally or alternatively be textured so that one or more indentations, hollows, protrusions, or roughed portions having various shapes and sizes may be positioned on or into a surface of the fabric 202. The indentations, hollows, protrusions, or roughed portions may not extend through the fabric 202. A surface of the fabric 202 may include a pattern or arrangement of particles or constituent parts of the fabric 202. In yet another example embodiment, a surface of the tissue fabric 202 may include any flexible or rigid protrusions extending from one side of the fabric 202. Such patterns or protrusions may have a variety of shapes such as, for example, pyramids, cylinders, ribs, or other non-symmetrical shapes. The protrusions may be arranged in regular or irregular patterns, and the patterns themselves may be irregular and/or non-symmetrically formed on the surface of the fabric 202. The protrusions of the surface may be formed with either a course or fine grain surface. The surface may be formed by embossing methods that cover the surface of the fabrics 202 with patterns of protrusions raised above the surface of the fabric 202.

As illustrated in at least Figure 3, the tissue interface 108 may include a fabric 202 with one or more hollows or indentations to provide one or more channels 208. The channels 208 may be positioned or formed on a surface of the fabric 202. For example, when reduced pressure is applied through the tissue interface 108, the channels 208 may direct or guide fluid along a surface of the fabric 202. The channels 208 may be positioned or formed randomly on the fabric 202 in an irregular pattern. In various embodiments, the channels 208 may be formed by embossing the fabric 202 (for example using embossing rollers), pressing a pattern of channels into the surface of the fabric 202, vacuum forming the fabric 202, or by any other method known by those skilled in the art. In some embodiments, portions of the fabric 202 may be stiffened so that channels 208 may be formed and maintained on a surface of the fabric 202. The channels 208 may have a variety of different shapes and sizes positioned where formed on the surface of the fabric 202. The channels 208 may have a linear shape, a curved shape, or an angular shape formed on a surface of the fabric 202. The channels 208 may have a cross-sectional shape including at least one of a semicircular shape, and popular shape, a V-shape, circle, or any other geometric shape known by those skilled in the art. The channels 208 may have a depth between about 200 µm and about 1000 µm and a width between about 200 µm and about 1000 µm. In various embodiments, at least one of the channels 208 may be positioned or formed on the surface of the fabric 202 to intersect with other features described herein including at least one of a perforation 204 or another channel 208. In at least some embodiments, the channels 208 may be positioned on a surface of the fabric 202 so that some of the channels 208 are more concentrated in at least one region of the fabric 202 while other channels 208 are less concentrated in another region of the fabric 202. In various embodiments, a channel 208 may be sized, shaped, or positioned on a surface of the fabric 202 such that the channel 208 cannot fold over itself or a separate layer or fabric of the tissue interface 108 and occlude another channel 208.

As illustrated in at least Figure 3, the tissue interface 108 may include a fabric 202 having hollows or indentations to provide one or more dimples 210. The dimples 210 may be positioned or formed on a surface of the tissue interface 108. The dimples 210 may create more surface area on the surface of the tissue interface 108 to facilitate absorption of fluids into pores of the tissue interface 108. The tissue interface 108 may be textured to form the one or more dimples 210 by pressing a pattern of dimples 210 into the surface of the tissue interface 108, by vacuum forming a pattern of dimples 210 into the surface of the tissue interface 108, or by any other method known by those skilled in the art. At least one of the dimples 210 may have a diameter between about 50 µm and about 2000 µm. The dimples 210 may have a hollow shape including the shape of a cone, an ellipsoid, a hemisphere, or a polyhedron. The dimples 210 also may have a depth between about 200 µm and about 1000 µm. The dimples 210 may have a variety of different shapes and sizes positioned where formed on the surface of the tissue interface 108. The dimples 210 may be positioned or formed randomly on the tissue interface 108 in an irregular pattern. In at least some embodiments, the dimples 210 may be positioned so that some of the dimples 155 are more concentrated in at least one region of the tissue interface 108 while other dimples 155 are less concentrated in another region of the tissue interface 108. In some embodiments, portions of the fabric 202 may be stiffened so that dimples 210 are maintained on a surface of the fabric 202. In various embodiments, a dimples 210 may be sized, shaped, or positioned on a surface of the tissue interface 108 such that the dimple 210 cannot fold over itself or a separate layer of the tissue interface 108 and occlude a channel 208 or another dimple 210.

As discussed herein, the fabric 202 may include at least one of a mesh, a mat, or spacer fabric. In some embodiments, the fabric 202 may be a mesh formed from one or more fibers 206 of the plurality of fibers 206 woven together to form the fabric 202. In some embodiments, the fabric 202 may be a mat formed from one or more fibers 206 of the plurality of fibers 206 pressed together to form the fabric 202. In some embodiments, the fabric 202 may be a spacer fabric formed from one or more fibers 206 of the plurality of fibers 206 in a thicker three-dimensional shape to form or maintain a space between two or more other elements, such as the cover 106 and a tissue site as discussed with respect to Figure 1.

Figure 4 is a flow chart of a method 400 for manufacturing a fabric of a tissue interface for the therapy system 100 of Figure 1. At step 405, a fabric of a tissue interface may be formed. The fabric may be the same as or similar to the fabric 202 of at least Figures 2 and 3. The tissue interface may be the same as or similar to the tissue interface 108 of at least Figure 1. The fabric 202 may include a plurality of fibers, such as fibers 206 illustrated in Figures 2 and 3. The fibers 206 may be extracted from a polymeric material, for example, by using an extrusion method. The polymeric material may also include at least one of a polymeric film or a non-woven material. The fibers 206 may be coupled, meshed, woven, bonded, or fused together as discussed herein. At step 410, the fabric 202 of the tissue interface 108 may be perforated or fenestrated to form one or more perforations 204. In at least some embodiments, the fabric 202 may be stretched to change a shape of the perforations 204. At step 415, the fabric 202 may be formed to include one or more channels 208. At step 420, a hydrophilic material may be applied to the fabric 202. The hydrophilic material may include at least one of a coating or a hydrophilic adhesive. At step 425, the fabric 202 may be fused with one or more fusing fibers. At step 430, the fabric 202 may be coated with an expanded polymer. At step 435, an absorbent layer may be disposed over the fabric 202.

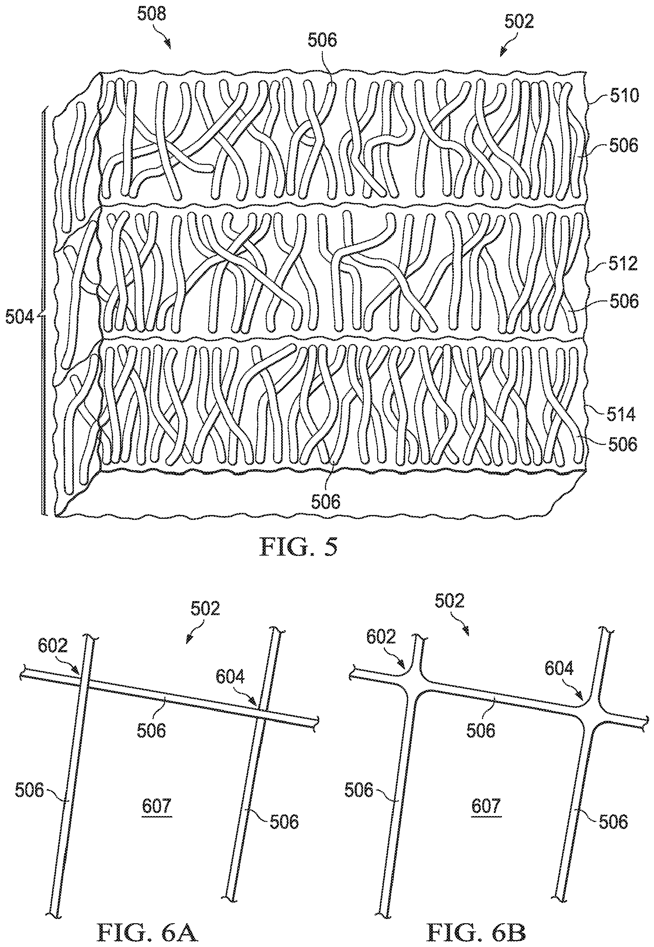

Turning to at least Figure 5, a tissue interface 508 may have a fabric 502 with one or more layers 504. The tissue interface 508 may be the same as or similar to the tissue interface 108 illustrated in Figures 1, 2, and 3. The fabric 502 may be the same as or similar to fabric 202 of Figures 2 and 3. Each of the layers 504 of the fabric 502 may include a plurality of fibers 506. In some embodiments, the plurality of fibers 506 may be the same as or similar to the plurality of fibers 206 of Figures 2 and 3. Each of fibers 506 may include a same material or various different materials. Further, the fibers 506 of a first layer may have a same material as the fibers 506 of a second layer or the fibers 506 of a first layer may have different materials than the fibers 506 of a second layer. As shown in Figure 5, the one or more layers 504 may include at least a top layer 510, a middle layer 512 (such as an inter-surface layer), and a bottom layer 514. The top layer 510 and the bottom layer 514 may include fibers 506 the permit flexing of the fabric 502. The middle layer 512 may include fibers 506 that are more elastic than the fibers 506 of the top layer 510 and the bottom layer 514. In some embodiments, the middle layer 512 may have a higher concentration of fibers 506 than the top layer 510 and the bottom layer 514.

Figure 6A is a perspective view of a first embodiment of at least some of the plurality of fibers 506 of the tissue interface 508 of Figure 5 contacting each other. As shown in Figure 6A, each of the fibers 506 may be in physical contact with each other or placed in close proximity to each other at fiber cross-over points 602 and 604. Figure 6B is a perspective view of a second embodiment of at least some of the plurality of fibers 506 of the tissue interface 508 of Figure 5 coupled to each other. As shown in Figure 6B, each of the fibers 506 may be coupled to each other at the fiber cross-over points 602 and 604. It should be understood that the term "coupled" may include bound, fastened, stuck, glued, welded, or securely touching.

After the fibers 506 are knitted or woven to form the fabric 502, the fibers 506 may be coupled to each other, put in direct physical contact with each other, or placed in close proximity to each other at the fiber cross-over points. As shown in Figure 6A, fibers 506 may cross-over each other at the fiber cross-over points 602 and 604. While the fibers 506 shown in Figure 6A may be in direct physical contact with each other or may come close to being in contact with each other at the fiber cross-over points 602 and 604, the fibers 506 may be able to move freely as they are not coupled to each other. As shown in Figure 6B, the fibers 506 may be coupled to each other at fiber cross-over points 602 and 604. Thus, in the example embodiment of Figure 6B, the fibers 506 may be unable to move freely at the fiber cross-over points 602 and 604. The fibers 506 may be coupled to each other at fiber cross-over points 602 and 604, for example, to stop or reduce shedding when the fabric 502 is cut or removed from a wound. Fibers 506 may be coupled to each other through a plurality of means including at least one of using an adhesive, heating, melting, fusing, twisting, tying, compressing, or other methods know by those having ordinary skill in the art. In some embodiments, after the fibers 506 are coupled to each other, a coating (such as a solvent coating, a water-based coating, or a foam-based coating) may be applied to the fibers 206 that upon drying deposits a thin, strong elastic polymer film on the fibers 506.

In some embodiments, when the fibers 506 are coupled to each other, one or more voids or gaps 607 may be formed between the fibers 506 to communicate fluid through the fabric 502. In various embodiments, coupling the fibers 506 at the fiber cross-over points 602 and 604 may generate an imperfect coupling between fibers 506. An imperfect coupling provides that some portions of a fiber 506 are coupled to another fiber 506 while other portions of the same fiber 506 are not coupled to the same fiber 506 or another fiber 506. Thus, imperfect coupling may provide portions of a fiber 506 that are free or not coupled to another fiber 506 to form the gaps 607 between fibers 506.

In at least some embodiments, the fiber cross-over points 602 and 604 may be positioned or may occur randomly at different locations along a particular fiber 506. Varying locations of the fiber cross-over points 602 and 604 may create varying sized gaps 607 through the fabric 502. For example, a fiber 506 that has two fiber cross-over points with other fibers 506 that are a first distance between each other may create a larger gap 607 through the fabric 502 than a fiber that has two fiber cross-over points with other fibers 506 that are a second and shorter distance between each other than the first distance. It should be understood that while Figures 6A and 6B illustrate a fiber 206 having two fiber cross-over points, a fiber 506 may have one or a plurality of cross-over points.

It should also be understood that the concentration of the fibers or fiber density in the fabric 502 may affect a size of the gaps 607 and the quantity of the gaps 607 through the fabric 502. For example, a first portion of the fabric 502, such as a first layer of the fabric 502, may have a greater concentration of fibers 506 than a second portion of the fabric 502, such as a second layer of the fabric 502. Accordingly, the first portion of the fabric 502 may have more fiber cross-over points on a particular fiber 506 than a fiber 506 of the second portion of the fabric 502. The more fiber cross-over points that exist on a particular fiber 506, the shorter the distance that may exist between fiber cross-over points on that particular fiber 506. Thus, the first portion of the fabric 502 may have gaps 607 with smaller cross-sectional areas than the gaps 607 of the second portion of the fabric 502. Furthermore, as the number of fiber cross-over points on a particular fiber 506 increases, so may the number of gaps 607 in the particular portion of the fabric 502. Thus, for example, the first portion of the fabric 502 may have more gaps 607 than the second portion of the fabric 502 because the fibers 506 of the first portion have more fiber cross-over points than the fibers 506 of the second portion.

In some embodiments, the fibers 506 may be coupled together using heat. The heat may form a temperature gradient through the fabric 502 that may also affect a size of the gaps 607 and a quantity of gaps 607 through the fabric 502. For example, after the fabric 502 is formed and the fabric 502 passes through a heating process that sets the fibers to reduce shrinking, the fibers 506 located in layers closer to a surface of the fabric 502 may experience more melting than the fibers 506 located further from the surface or closer to a center of the fabric 502. This may be due to a reliance on a heat source that is external to the fabric 502 to transfer heat to and creating a temperature gradient through the fabric 502. Because the fibers 506 that are closer a surface of the fabric 502 may experience more melting, more fibers that are closer to the surface of the fabric 502 may be coupled at more fiber locations than fibers that are further from a surface of the fabric 502 or closer to a center of the fabric 502. The temperature gradient through the fabric 502 may create more gaps 607 and gaps 607 with smaller cross-sectional areas in locations of the fabric 502 that are closer to a surface of the fabric 502 relative to locations of the fabric 502 that are further from the surface of the fabric 502 or closer to a center of the fabric 502. Accordingly, the greater the temperature gradient through the fabric 502, the greater the amount of gaps 607 and the smaller the cross-sectional area of each of the gaps 607 are in areas closers to a surface of the fabric 502 or closer to a heat source relative to the number of gaps 607 and the size of the gaps 607 in areas closer to a center of the fabric 502 or further from the heat source.

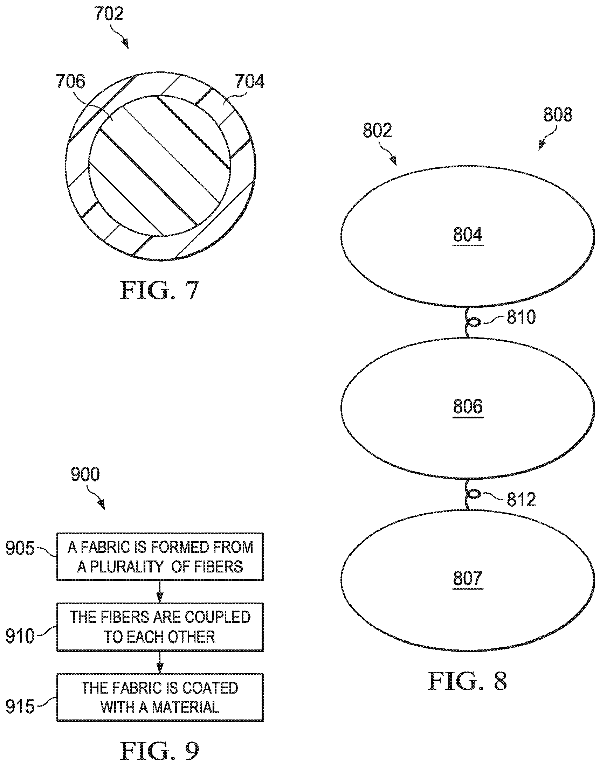

In various embodiments, one or more fibers 506 of the plurality of fibers 506 of Figures 5, 6A, and 6B may be single component fibers. Single component fibers may be fibers that include only one material or yarn. In some embodiments, one or more fibers 506 of the plurality of fibers 506 of Figures 5, 6A, and 6B may be bicomponent fibers. Bicomponent fibers may include fibers that include two different materials or yarns. Figure 7 is a cross-sectional view illustrating details that may be associated with some example embodiments of the fibers 506 of Figures 5, 6A, 6B. Specifically, Figure 7 illustrates an example embodiment of a bicomponent fiber 702. As shown in Figure 7, a bicomponent fiber 702 may include an outer layer or outer sheath layer 704 and an inner layer or inner core 706. The outer sheath layer 704 may be made of a first material and the inner core 706 may be made of a second and different material. It should be understood that the fibers 506 of a tissue interface 508 may all be bicomponent fibers or a combination of bicomponent fibers and single component fibers.

In some embodiments, a biocomponent fiber 702 may include a first material and a second material, where the first material that has a lower melting point than the second material. For example, a biocomponent fiber 702 may include an outer sheath layer 704 made of a first material that has a lower melting point than a second material forming the inner core 706. Thus, after the fabric 502, that includes biocomponent fibers 702, is formed, the fabric 502 may pass through a heating process that sets the fibers to reduce shrinking. The heating process may reach a temperature that also melts at least a portion of the outer sheath layer 704 of the biocomponent fibers 702 without melting the inner core 706 of each of the bicomponent fibers 702. The lower melting point of the first material of the outer sheath layer 704 relative to the higher melting point of the second material of the inner core 706 may allow the outer sheath layer 704 to melt within a range of temperatures without melting the inner core 706. One of ordinary skill in the art would be able to identify a range of temperatures that may melt a first material of an outer sheath layer 704 without melting a second material of an inner core 706. As the outer sheath layer 704 melts, the bicomponent fibers 702 may couple with other fibers including single component fibers or other bicomponent fibers 702 at fiber cross-over points where fibers come into close contact or direct physical contact.

Figure 8 is a perspective schematic view illustrating a second embodiment of a tissue interface for the therapy system 100 of Figure 1. The tissue interface illustrated in Figure 8 may be the same as or similar to the tissue interface 108 illustrated in Figure 1 or the tissue interface 508 illustrated in Figure 5. For example, the tissue interface 808 may include one or more features of the tissue interface 108 of Figure 1. In the example embodiment of Figure 8, the tissue interface 808 may include a fabric 802. The fabric 802 may be the same as or similar to fabric 202 of Figures 2 and 3 or the fabric 502 of Figure 5. In some embodiments, after the fabric 802 is formed, the fabric 802 may be coated with a strongly elastic polymer coating such as a polyurethane dispersion (PUD) coating. Working examples of PUD coatings include PUD coatings available from BAYER^{™}. The fabric 802 may also be pre-cut to size where the edges are fused and sealed preventing or reducing shedding. In some embodiments, the pre-cut fabric may be cut into separate and various sizes. In some embodiments, the edges of the pre-cut fabric may be stitched, for example using an overlocking stitching technique that encapsulates any free fibers with the fabric 802. In some embodiments, the edges of the pre-cut fabric may be fused using hot cutting blades, ultrasonic fusing, or radio frequency fusing. As shown in the example embodiment of Figure 8, the fabric 802 may be cut into one or more fabric pieces, such as a first fabric piece 804, a second fabric piece 806, and a third fabric piece 807. Each piece of the one or more fabric pieces may be linked to each other using fibers 810 and 812. The fibers 810 and 812 may be cut by a user to place the individual fabric pieces into a wound or on a tissue site.

Figure 9 is a flow chart of a second method 900 of manufacturing a tissue interface for the therapy system of Figure 1. At step 905, a fabric of a tissue interface may be formed. The fabric may be the same as or similar to the fabric 202 of Figures 2 and 3, the fabric 502 of Figure 5, or the fabric 802 of Figure 8. The tissue interface may be the same as or similar to the tissue interface 108 of Figure 1, the tissue interface 508 of Figure 5, or the tissue interface 808 of Figure 8. The fabric may include a plurality of fibers, such as fibers 206 of Figures 2 and 3, the fiber 506 of Figure 5, or the biocomponent fibers 702 of Figure 7. At step 910, each of the fibers may be coupled to each other at one or more fiber cross-over points. At step 915, the fabric may be coated with a material such as a solvent or a foam-based material that upon drying deposits thin, strong, and elastic polymer film on the fibers. The effective gap size of the fabrics may be individually different due to, for example, the concepts discussed herein. As such, fabrics may have both large and small gaps sizes on the surfaces of the tissue interface 808 (such as on a top layer and a bottom layer). A fabric with large gaps on the surfaces could be used in wound contact where granulation is desired. A fabric with small gaps could be used where little or no granulation is desired. The fabric could also be structured to give a range of apposition forces in different directions depending on the orientation that was used when placing the fabric in a wound or at a tissue site. The fabrics, as discussed herein, may be significantly stronger than GRANUFOAM^{™} and gauze while at least reducing a risk of leaving fragments in a wound upon removal of the fabric due to an increase in fabric strength. In some embodiments, the fabrics discussed herein may include a radiopaque material permitting x-ray location. In some embodiments, fibers as discussed herein may include antimicrobial agents, such as silver.

The systems, apparatuses, and methods described herein may provide significant advantages. For example, the tissue interface (such as wound filler) may reduce trauma and facilitate ease of removal when treating both deep and shallow wounds. The tissue interface encourages granulation without the disadvantage of tissue ingrowth resulting in pain or discomfort upon removal.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the container 112, or both may be eliminated or separated from other components for manufacture or sale. In other example configurations, the controller 110 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

Aspects of the invention are disclosed in the following numbered clauses:
1. A method of manufacturing a tissue interface for a reduced-pressure tissue treatment system, the method comprising:
   forming a fabric comprising a plurality of fibers, wherein each of the plurality of fibers comprises a polymeric material;
   coupling the plurality of fibers of the fabric together; and
   applying a hydrophilic material to the plurality of fibers.
2. The method of Clause 1, wherein the plurality of fibers are one of bicomponent fibers or single component fibers.
3. The method of Clause 1, further comprising coating the plurality of fibers with a coating that comprises at least one of a solvent based coating, a water based coating, or a solventless liquid coating.
4. The method of Clause 3, wherein the coating is an elastic polymer coating.
5. The method of Clause 3, wherein the elastic polymer coating comprises a polyurethane dispersion (PUD) coating.
6. The method of Clause 1, wherein each of the plurality of fibers comprises an outer sheath material formed over an inner material, and wherein the outer sheath material comprises a lower melting point than the inner material.
7. The method of Clause 6, wherein coupling the plurality of fibers comprises melting an outer sheath material of at least one of the plurality of fibers without melting the inner material of each of the plurality of fibers.
8. The method of Clause 1, wherein forming the fabric comprises forming a fabric comprising a top layer, an inter-surface layer, and a bottom layer.
9. The method of Clause 8, wherein at least one of the top layer, the inter-surface layer, or the bottom layer comprise at least one fiber that is different from one or more remaining fibers of the plurality of fibers of the fabric.
10. The method of Clause 1, wherein at least one of the plurality of fibers comprises at least one of a woven, a non-woven, or a knitted material.
11. The method of Clause 10, wherein a thickness of the woven, non-woven, or knitted material is between approximately 1 millimeter (mm) and approximately 30 mm.
12. The method of Clause 1, wherein coupling the plurality of fibers comprises heating the plurality of fibers.
13. The method of Clause 1, wherein the fabric comprises a thickness between approximately 1 mm and approximately 2 mm.
14. The method of Clause 1, wherein the fabric comprises one or more perforations.
15. The method of Clause 14, wherein a diameter or width of each of the one or more perforations is between approximately 3 mm and approximately 2 mm.
16. The method of Clause 1, wherein a first set of fibers of the plurality of fibers forms at least one of a top layer of the fabric or a bottom layer of the fabric.
17. The method of Clause 16, wherein the first set of fibers comprises a first density, and wherein the first set of fibers is configured to permit flexibility of the fabric.
18. The method of Clause 17, wherein a second set of fibers of the plurality of fibers forms an inter-surface layer of the fabric, and wherein the second set of fibers comprises a second density that is greater than the first density.
19. The method of Clause 1, further comprising configuring the fabric to be positioned at or within a wound.
20. The method of Clause 19, wherein configuring the fabric comprises trimming the fabric.
21. The method of Clause 19, further comprising fusing one or more edges of the fabric after configuring the fabric to be positioned at or within the wound using at least one of hot cutting blades, ultrasonic techniques, or radio frequency techniques.
22. The method of Clause 1, wherein the fabric is configured to retain free fibers.
23. The method of Clause 22, wherein edges of the fabric are overlocked to retain the free fibers
24. The method of Clause 23, wherein the edges of the fabric are overlocked using stitching.
25. A system to provide reduced pressure at a tissue site, the system comprising:
   a cover configured to form a seal over the tissue site;
   a reduced-pressure source configured to provide reduced pressure through the cover at the tissue site; and
   a tissue interface configured to be disposed underneath the cover at the tissue site and comprising a fabric having plurality of fibers that are coupled together.
26. The system of Clause 25, wherein the plurality of fibers are one of bicomponent fibers or single component fibers.
27. The system of Clause 25, wherein each of the plurality of fibers is coated with a coating comprising at least one of a solvent based coating or a water based coating.
28. The system of Clause 27, wherein the coating is an elastic polymer.
29. The system of Clause 28, wherein the elastic polymer comprises a polyurethane dispersion (PUD) coating.
30. The system of Clause 29, wherein each of the plurality of fibers comprises an outer sheath material formed over an inner material, and wherein the outer sheath material comprises a lower melting point than the in Clause terial.
31. The system of Clause 30, wherein the plurality of fibers are fused together by melting the outer sheath material of at least one of the plurality of fibers without melting the inner material of each of the plurality of fibers.
32. The system of Clause 25, wherein the fabric comprises a top layer, an inter-surface layer, and a bottom layer.
33. The system of Clause 32, wherein at least one of the top layer, the inter-surface layer, or the bottom layer comprise at least one fiber that is a different material from one or more remaining fibers of the plurality of fibers of the fabric.
34. The system of Clause 25, wherein at least one of the plurality of fibers comprises at least one of a woven, a non-woven, or a knitted material.
35. The system of Clause 34, wherein a thickness of at least one of the woven, the non-woven, or the knitted material is between approximately 1 millimeter (mm) and approximately 30 mm.
36. The system of Clause 25, wherein the plurality of fibers are coupled together by heating the plurality of fibers.
37. The system of Clause 25, wherein the fabric comprises a thickness between approximately 1 mm and approximately 2 mm.
38. The system of Clause 25, wherein the fabric comprises one or more perforations.
39. The system of Clause 38, wherein a diameter or width of each of the one or more perforations is between approximately 3 mm and approximately 2 mm.
40. The system of Clause 25, wherein a first set of fibers of the plurality of fibers forms at least one of a top layer of the fabric or a bottom layer of the fabric.
41. The system of Clause 40, wherein the first set of fibers comprises a first density, and wherein the first set of fibers is configured to permit flexibility of the fabric.
42. The system of Clause 41, wherein a second set of fibers of the plurality of fibers forms an inter-surface layer of the fabric, and wherein the second set of fibers comprises a second density that is greater than the first density.
43. The system of Clause 25, wherein the fabric is configured to be positioned at or within a wound.
44. The system of Clause 43, wherein the fabric is configured to be positioned at or within the wound by trimming the fabric.
45. The system of Clause 25, wherein one or more edges of the fabric are fused together using at least one of hot cutting blades, ultrasonic techniques, or radio frequency techniques.
46. The system of Clause 25, wherein the fabric is configured to retain free fibers.
47. The system of Clause 46, wherein edges of the fabric are overlocked to retain the free fibers.
48. The system of Clause 47, wherein the edges of the fabric are overlocked using stitching.
49. A method of manufacturing a tissue interface for a reduced-pressure tissue treatment system, the method comprising:
   forming a mesh comprising a plurality of strands meshed together, wherein each of the plurality of strands comprises a polymeric film;
   perforating the mesh to form a perforated mesh having one or more perforations; and
   texturing the perforated mesh to form a textured and perforated mesh having at least one textured surface.
50. The method of Clause 49, wherein the polymeric film comprises a non-woven material.
51. The method of Clause 49, wherein texturing the perforated mesh to form the textured and perforated mesh having at least one textured surface comprises at least one of embossing or vacuum forming the perforated mesh.
52. The method of Clause 49, further comprising after perforating the mesh, stretching the perforating mesh to change a shape of the one or more perforations.
53. The method of Clause 49, wherein a shape of the one or more perforations comprises at least one of a circle, a polygon, or an oval.
54. The method of Clause 49, wherein the one or more perforations is between approximately 3 mm and approximately 2 mm.
55. The method of Clause 49, wherein a cross-sectional shape of each of the plurality of strands comprises at least one of a circle, a polygon, or an oval.
56. The method of Clause 49, further comprising applying a hydrophilic treatment to mesh.
57. The method of Clause 56, wherein the hydrophilic treatment comprises at least one of a coating or a hydrophilic adhesive.
58. The method of Clause 49, wherein mesh comprises one or more pores each having a diameter or width between approximately 300 µm (microns) and approximately 2000 µm.
59. The method of Clause 49, wherein the plurality of strands is meshed together by bonding at least one strand of the plurality of strand together with at least one other strand of the plurality of strands using at least one of a weld or a glue.
60. The method of Clause 49, wherein the textured surface comprises at least one of a dimple or a channel.
61. The method of Clause 60, wherein a diameter of the dimple is between approximately 50 µm and approximately 2000 µm.
62. The method of Clause 60, where a shape of the dimple comprises at least one of a cone, an ellipsoid, a spherical cap, or a polyhedron.
63. The method of Clause 60, wherein a depth of the channel is between approximately 50 µm and approximately 1000 µm.
64. The method of Clause 60, wherein a width of the channel is between approximately 50 µm and approximately 1000 µm.
65. The method of Clause 49, further comprising fusing mesh with one or more fibers.
66. The method of Clause 49, further comprising coating mesh with an expanded polymer.
67. The method of Claim 49, further comprising disposing an absorbent layer over the mesh.
68. A system to provide reduced pressure at a tissue site, the system comprising:
   a cover configured to form a seal over the tissue site;
   a reduced-pressure source configured to provide reduced pressure through the cover at the tissue site; and
   a tissue interface configured to be disposed underneath the cover at the tissue site and comprising a textured mat having a plurality of strands of polymeric film matted together and at least one textured surface.
69. The system of Clause 68, wherein the polymeric film comprises a non-woven material.
70. The system of Clause 68, wherein the mat is textured using at least one of vacuum forming or embossing.
71. The system of Clause 70, wherein the mat is perforated to form one or more perforations.
72. The system of Clause 71, wherein a shape of the one or more perforations is formed by stretching the perforated mat after the one or more perforations are formed.
73. The system of Clause 71, wherein a shape of the one or more perforations comprises at least one of a circle, a polygon, an oval, a slot, or a slit.
74. The system of Clause 71, wherein a diameter or width of the one or more perforations is between approximately 3 mm and approximately 2 mm.
75. The system of Clause 68, wherein the plurality of strands are extruded from one or more polymeric films.
76. The system of Clause 75, wherein at least one strand of the plurality of strands comprises a polymeric film that is different from a polymeric film of at least one other strand of the plurality of strands.
77. The system of Clause 75, wherein a cross-sectional shape of each of the plurality of strands comprises at least one of a circle, a polygon, an oval, a slot, or a slit.
78. The system of Clause 75, wherein the plurality of strands is bonded with at least one of a weld or a glue.
79. The system of Clause 68, wherein the tissue interface further comprises a hydrophilic treatment comprising at least one of a coating or a hydrophilic adhesive.
80. The system of Clause 68, wherein mat comprises one or more pores each having a diameter or width between approximately 300 µm (microns) and approximately 2000 µm.
81. The system of Clause 68, wherein the textured surface comprises at least one of a dimple or a channel.
82. The system of Clause 81, wherein a diameter of the dimple is between approximately 50 µm and approximately 2000 µm.
83. The system of Clause 81, wherein a shape of the dimple comprises at least one of a cone, an ellipsoid, a spherical cap, or a polyhedron.
84. The system of Clause 81, wherein a depth of the channel is between approximately 50 µm and approximately 1000 µm.
85. The system of Clause 81, wherein a width of the channel is between approximately 50 µm and approximately 1000 µm.
86. The system of Clause 68, wherein each of the plurality of strands is fused with one or more fusing fibers.
87. The system of Clause 68, wherein the mat is coated with an expanded polymer.
88. The system of Clause 68, wherein the tissue interface comprises an absorbent layer disposed over the mat.
89. A tissue interface configured to be disposed at a tissue site, the tissue interface comprising:
   a textured mat comprising:
      a plurality of matted strands of polymeric film, and
      at least one of a dimple or a channel; and
   wherein the tissue interface is configured to:
      be disposed underneath a cover at the tissue site, and
      receive reduced pressure from a reduced pressure source.
90. The tissue interface of Clause 89, wherein the polymeric film comprises a non-woven material.
91. The tissue interface of Clause 89, wherein the mat is textured using at least one of vacuum forming or embossing.
92. The tissue interface of Clause 89, wherein the mat is perforated to form one or more perforations.
93. The tissue interface of Clause 92, wherein a shape of the one or more perforations is formed by stretching the textured mat.
94. The tissue interface of Clause 92, wherein a shape of the one or more perforations comprises at least one of a circle, a polygon, an oval, a slot, or a slit.
95. The tissue interface of Clause 92, wherein a diameter or width of the one or more perforations is between approximately 3 mm and approximately 2 mm.
96. The tissue interface of Clause 89, wherein the plurality of strands are extruded from one or more polymeric films.
97. The tissue interface of Clause 96, wherein at least one strand of the plurality of strands comprises a polymeric film that is different from a polymeric film of at least one other strand of the plurality of strands.
98. The tissue interface of Clause 96, wherein a cross-sectional shape of each of the plurality of strands comprises at least one of a circle, a polygon, an oval, a slot, or a slit.
99. The tissue interface of Clause 96, wherein the plurality of strands is bonded with at least one of a weld or a glue.
100. The tissue interface of Clause 90, wherein the tissue interface further comprises a hydrophilic treatment comprising at least one of a coating or a hydrophilic adhesive.
101. The tissue interface of Clause 100, wherein mat comprises one or more pores each having a diameter or width between approximately 300 µm (microns) and approximately 2000 µm.
102. The tissue interface of Clause 100, wherein the textured mat comprises one or more textured surfaces, wherein at least one textured surface comprises at least one of a dimple or a channel.
103. The tissue interface of Clause 102, wherein a diameter of the dimple is between approximately 50 µm and approximately 2000 µm.
104. The tissue interface of Clause 102, wherein a shape of the dimple comprises at least one of a cone, an ellipsoid, a spherical cap, or a polyhedron.
105. The tissue interface of Clause 102, wherein a depth of the channel is between approximately 50 µm and approximately 1000 µm.
106. The tissue interface of Clause 102, wherein a width of the channel is between approximately 50 µm and approximately 1000 µm.
107. The tissue interface of Clause 119, wherein the each of the plurality of strands is fused with one or more fusing fibers.
108. The tissue interface of Clause 89, wherein the texture mat is coated with an expanded polymer.
109. The tissue interface of Clause 89, wherein the tissue interface comprises an absorbent layer disposed over the textured mat.
110. A tissue interface configured to be disposed at a tissue site, the tissue interface comprising:
   a spacer fabric having a top layer, a middle layer, and a bottom layer, wherein each of the top layer, the middle layer, and the bottom layer comprises a plurality of fibers, wherein the plurality of fibers is coupled together by melting an outer sheath of each of the plurality of fibers without melting an inner material of each of the plurality of fibers, wherein the outer sheath is formed over the inner material of each of the plurality of fibers, wherein the outer sheath comprises a melting point temperature that is lower than a melting point temperature of the inner material, wherein each of the plurality of fibers is coated with a material comprising at least one of a solvent based material, a water based material, or a solventless liquid material, wherein the material is an elastic polymer, and wherein the elastic polymer comprises a polyurethane dispersion (PUD) material.
111. A tissue interface configured to be disposed at a tissue site, the tissue interface comprising:
   a textured and perforated mat comprising:
      a plurality of matted strands of polymeric film, wherein at least one strand of the plurality of strands comprises a polymeric film that includes different material from a material of a polymeric film of at least one other strand of the plurality of strands,
      at least one of a dimple or a channel formed on a surface of the mat, and
      one or more perforations formed by perforating the mat, wherein a shape of the one or more perforations comprises at least one of a circle, a polygon, or an oval, and wherein a diameter or width of the one or more perforations is between approximately 3 mm and approximately 2 mm; and
   wherein the tissue interface is configured to:
      be disposed underneath a cover at the tissue site, and
      receive reduced pressure from a reduced pressure source.

## Claims

1. A method of manufacturing a tissue interface for a reduced-pressure tissue treatment system, the method comprising:
forming a mesh comprising a plurality of strands meshed together, wherein each of the plurality of strands comprises a polymeric film;
perforating the mesh to form a perforated mesh having one or more perforations; and
texturing the perforated mesh to form a textured and perforated mesh having at least one textured surface.

2. The method of Claim 1, wherein the polymeric film comprises a non-woven material.

3. The method of Claim 1, wherein texturing the perforated mesh to form the textured and perforated mesh having at least one textured surface comprises at least one of embossing or vacuum forming the perforated mesh.

4. The method of Claim 1, further comprising applying a hydrophilic treatment to mesh.

5. The method of Claim 4, wherein the hydrophilic treatment comprises at least one of a coating or a hydrophilic adhesive.

6. The method of Claim 1, wherein mesh comprises one or more pores each having a diameter or width between approximately 300 µm (microns) and approximately 2000 µm.

7. The method of Claim 1, further comprising disposing an absorbent layer over the mesh.

8. A system to provide reduced pressure at a tissue site, the system comprising:
a cover configured to form a seal over the tissue site;
a reduced-pressure source configured to provide reduced pressure through the cover at the tissue site; and
a tissue interface configured to be disposed underneath the cover at the tissue site and comprising a textured mat having a plurality of strands of polymeric film matted together and at least one textured surface.

9. The system of Claim 8, wherein the polymeric film comprises a non-woven material.

10. The system of Claim 9, wherein the mat is textured using at least one of vacuum forming or embossing, and is optionally perforated to form one or more perforations.

11. The system of Claim 8, wherein the plurality of strands are extruded from one or more polymeric films.

12. The system of Claim 11, wherein at least one strand of the plurality of strands comprises a polymeric film that is different from a polymeric film of at least one other strand of the plurality of strands.

13. The system of Claim 8, wherein the tissue interface further comprises a hydrophilic treatment comprising at least one of a coating or a hydrophilic adhesive.

14. The system of Claim 8, wherein each of the plurality of strands is fused with one or more fusing fibers.

15. The system of Claim 8, wherein the mat is coated with an expanded polymer.
